# EUROPÄISCHE PATENTSCHRIFT

(11) **EP 3 272 759 B1**
(45) Veröffentlichungstag und Bekanntmachung des Hinweises auf die Patenterteilung: **08.05.2019**
(21) Anmeldenummer: 16180055.2
(22) Anmeldetag: 19.07.2016
(51) Int. Cl.: C07C 67/38, C07F 15/00, C07F 17/02

(54) **1,1'-BIS(PHOSPHINO)FERROCENLIGANDEN FÜR DIE ALKOXYCARBONYLIERUNG**
1,1 -BIS (PHOSPHINO) FERROCENE LIGANDS FOR ALKOXYCARBONYLATION
LIGANDS DE 1,1 -BIS(PHOSPHINO)FERROCÈNE POUR L'ALKOXY-CARBONYLATION

(43) Veröffentlichungstag der Anmeldung: 24.01.2018
(73) Patentinhaber: Evonik Degussa GmbH, 45128 Essen (DE)
(72) Erfinder: DONG, Kaiwu, 236800 Bo Zhou (CN); NEUMANN, Helfried, 18055 Rostock (DE); JACKSTELL, Ralf, 27478 Cuxhaven Altenwalde (DE); BELLER, Matthias, 18211 Ostseebad Nienhagen (DE); FRIDAG, Dirk, 45721 Haltern am See (DE); HESS, Dieter, 54770 Marl (DE); DYBALLA, Katrin Marie, 45657 Recklinghausen (DE); GEILEN, Frank, 45721 Haltern am See (DE); FRANKE, Robert, 45772 Marl (DE)

(56) Entgegenhaltungen:
- JP-A- 2000 256 384
- MALENZA F ET AL: "FERRROCENYL DIPHOSPHINES CONTAINING STEREOGENIC PHOSPHORUS ATOMS. SYNTHESIS AND APPLICATION IN THE RHODIUM-CATALYZED ASYMMETRIC HYDROGENATION", ORGANOMETALLICS, AMERICAN CHEMICAL SOCIETY, US, Bd. 18, 1. Januar 1999 (1999-01-01), Seiten 1041-1049, XP001040372, ISSN: 0276-7333, DOI: 10.1021/OM980754A
- TSURUTA H ET AL: "A new P-chiral bisphosphine, 1,1'-bis[(t-butyl)methyl-phosphino]ferroce ne, as an effective ligand in catalytic asymmetric hydrosilylation of simple ketones", TETRAHEDRON ASYMMETRY, PERGAMON PRESS LTD, OXFORD, GB, Bd. 10, Nr. 5, 12. März 1999 (1999-03-12), Seiten 877-882, XP004162510, ISSN: 0957-4166, DOI: 10.1016/S0957-4166(99)00042-7
- NETTEKOVEN U ET AL: "Phosphoris-Chiral Analogues of 1,1'-Bis(diphenylphosphino)ferrocene: Asymmetric Synthesis and Application in Highly Enantioselective Rhodium-Catalyzed Hydrogenation Reactions", THE JOURNAL OF ORGANIC CHEMISTRY, AMERICAN CHEMICAL SOCIETY, US, Bd. 64, 6. Mai 1999 (1999-05-06), Seiten 3996-4004, XP002377515, ISSN: 0022-3263, DOI: 10.1021/JO982481Z
- BRIAN J. ANDERSON ET AL: "Substrate and Catalyst Screening in Platinum-Catalyzed Asymmetric Alkylation of Bis(secondary) Phosphines. Synthesis of an Enantiomerically Pure C 2 -Symmetric Diphosphine", ORGANOMETALLICS, Bd. 27, Nr. 19, 13. Oktober 2008 (2008-10-13), Seiten 4992-5001, XP055298940, US ISSN: 0276-7333, DOI: 10.1021/om800534k
- NETTEKOVEN U ET AL: "PHOSPHORUS-CHIRAL DIPHOSPHINES AS LIGANDS IN HYDROFORMYLATION. AN INVESTIGATION ON THE INFLUENCE OF ELECTRONIC EFFECTS IN CATALYSIS", ORGANOMETALLICS, AMERICAN CHEMICAL SOCIETY, US, Bd. 19, Nr. 22, 30. Oktober 2000 (2000-10-30), Seiten 4596-4607, XP000975051, ISSN: 0276-7333, DOI: 10.1021/OM0004489
- BIANCHINI C ET AL: "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERRO CENE (DPPOMF)", ORGANOMETALLICS, AMERICAN CHEMICAL SOCIETY, US, Bd. 22, Nr. 12, 9. Juni 2003 (2003-06-09), Seiten 2409-2421, XP001164644, ISSN: 0276-7333, DOI: 10.1021/OM021049B

## Beschreibung

Die Erfindung betrifft ein Diastereomerengemisch von 1,1'-Bis(phosphino) ferrocenverbindungen, Metallkomplexe dieser Verbindungen und deren Verwendung für die Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefine) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

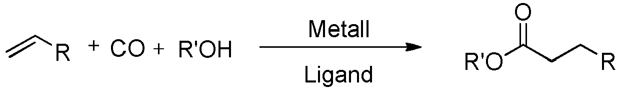

Unter den Alkoxycarbonylierungsreaktionen ist insbesondere die Reaktion von Ethen und Methanol zu 3-Methylpropionat (Ethen-Methoxycarbonylierung) von Bedeutung als Zwischenschritt für die Herstellung von Methylmethacrylat (S. G. Khokarale, E. J. Garcia-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Üblicherweise werden dabei zweizähnige Diphosphinverbindungen als Liganden eingesetzt. Ein sehr gutes katalytischen System wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

Anwendungen der Methoxycarbonylierung auf längerkettige Substrate sind z.B. in EP 0 662 467 beschrieben. Die Patentschrift beschreibt einen Prozess zur Herstellung von Dimethyladipat aus Methyl-3-Pentenoat. Als Pd-Quelle wird Pd(II)acetat verwendet. Als Beispiele geeigneter bidentater Phosphinliganden werden unter anderem 1,1'-Bis(diphenylphosphino)ferrocen, 1-(Diphenylphosphino)-1'-(diisopropylphosphino)ferrocen und 1,1'-Bis(isopropylphenylphosphino)ferrocen genannt. Die Liganden erzielen jedoch nur unzureichende Ausbeuten bei der Methoxycarbonylierung von Olefinen, insbesondere von langkettigen Olefinen wie 2-Octen und Di-n-Buten.

In BIANCHINI C ETAL, "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERROCENE (DPPOMF)", ORGANOMETALLICS, AMERICAN CHEMICAL SOCIETY, US, (20030609), Bd. 22, Nr. 12, Seiten 2409 - 2421, wird ein Verfahren zur Methoxycarbonylierung von Ethen beschrieben. Hierbei kommen die beiden folgenden P_{d}-Komplexe zum Einsatz: [Pd(H₂O)₂(dppf)]-(OTs)₂ und [Pd(H₂O)₂(dppomf)](OTs)₂.

Die vorliegende Erfindung hat die Aufgabe, neue Liganden für die Alkoxycarbonylierung bereitzustellen, mit denen sich gute Ausbeuten an Estern erzielen lassen. Insbesondere sollen die erfindungsgemäßen Liganden für die Alkoxycarbonylierung von langkettigen ethylenisch ungesättigten Verbindungen, beispielsweise C₈-Olefinen, und von Gemischen ethylenisch ungesättigter Verbindungen geeignet sein.

Diese Aufgabe wird gelöst durch ein Diastereomerengemisch von 1,1'-Bis(phosphino)ferrocenverbindungen, die an den zwei Phosphoratomen jeweils mit wenigstens einem Heteroarylrest substituiert sind, wobei das Gemisch zwei bestimmte Diastereomere umfasst. Dabei wurde gefunden, dass Palladiumkomplexe aus dem erfindungsgemäßen Diastereomerengemisch zu guten Ausbeuten bei der Alkoxycarbonylierung ethylenisch ungesättigter Verbindungen, insbesondere von C₈-Olefinen, führen. Auf eine Trennung der Diastereomere und die Herstellung von diastereomerenreinen Liganden kann somit verzichtet werden. Da eine solche Trennung immer sehr aufwändig und kostenintensiv ist, wird somit auch der Gesamtprozess wirtschaftlicher. Ferner fallen weniger Nebenprodukte an, da in diesem Fall eine Mischung der Diastereomeren verwendet werden kann und nicht eines der Diastereomeren entsorgt werden muss.

Das erfindungsgemäße Diastereomerengemisch umfasst Diastereomere gemäß den Formeln (**I.1**) und (**I.2**) 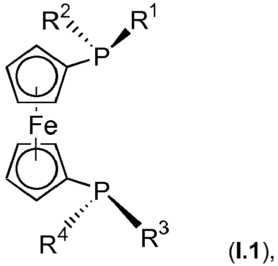 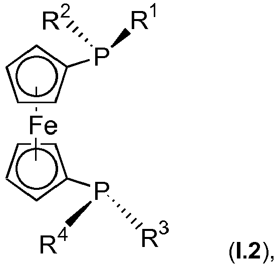 wobei
R², R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl;
die Reste R¹, R³ jeweils für einen -(C₃-C₂₀)-Heteroarylrest stehen;
R¹, R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, - COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl,-(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können;
   und
R², R⁴, falls sie für -(C₁-C₁₂)-Alkyl, -(C₃-C₁₂)-Cycloalkyl, -(C₃-C₁₂)-Heterocycloalkyl oder-(C₆-C₂₀)-Aryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl,-(C₃-C₂₀)-Heteroaryl-(C₁-C₁₂)-Alkyl, -(C₃-C₂₀)-Heteroaryl-O-(C₁-C₁₂)-Alkyl, -COOH, -OH,-SO₃H, -NH₂, Halogen substituiert sein können.

Es hat sich gezeigt, dass das erfindungsgemäße Diastereomerengemisch insbesondere dann zu einem wirksamen Katalysator führt, falls die beiden Diastereomere in einem bestimmten molaren Verhältnis vorliegen. So liegt das molare Verhältnis von (**I.1**) zu (**I.2**) vorzugsweise im Bereich von 10:90 bis 70:30, insbesondere von 20:80 bis 60:40, besonders bevorzugt von 30:70 bis 50:50, am meisten bevorzugt von 35:65 bis 45:55.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, *iso*-Butyl, *sec-*Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R³ jeweils unabhängig voneinander mit einem oder mehreren Substituenten ausgewählt aus -(C₁-C₁₂)-Alkyl, -(C₃-C₁₂)-Cycloalkyl,-(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R¹, R³ jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, substituiert sein.

In einer Ausführungsform können die Reste R¹, R³ jeweils unabhängig voneinander mit einem oder mehreren Substituenten ausgewählt aus -(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R¹, R³ jeweils unabhängig voneinander mit einem oder mehreren Substituenten ausgewählt aus -(C₁-C₁₂)-Alkyl und -(C₃-C₂₀)-Heteroaryl substituiert sein.

In einer Ausführungsform sind die Reste R¹, R³ unsubstituiert.

In einer Ausführungsform können die Reste R², R⁴, falls sie für -(C₁-C₁₂)-Alkyl, , -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R², R⁴, falls sie für -(C₁-C₁₂)-Alkyl, -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl,-(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, substituiert sein.

In einer Ausführungsform können die Reste R², R⁴, falls sie für -(C₁-C₁₂)-Alkyl, -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R², R⁴, falls sie für -(C₁-C₁₂)-Alkyl, -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl und-(C₃-C₂₀)-Heteroaryl substituiert sein.

In einer Ausführungsform sind die Reste R², R⁴ unsubstituiert, falls sie für -(C₁-C₁₂)-Alkyl, - (C₃-C₁₂)-Cycloalkyl oder -(C₃-C₁₂)-Heterocycloalkyl stehen, und können wie beschrieben substituiert sein, falls sie für -(C₆-C₂₀)-Aryl stehen.

In einer Ausführungsform sind die Reste R², R⁴ unsubstituiert, falls sie für -(C₁-C₁₂)-Alkyl,-(C₃-C₁₂)-Cycloalkyl, -(C₃-C₁₂)-Heterocycloalkyl oder -(C₆-C₂₀)-Aryl stehen.

Vorzugsweise sind R², R⁴ jeweils unabhängig voneinander ausgewählt aus -(C₁-C₁₂)-Alkyl,-(C₃-C₁₂)-Cycloalkyl, -(C₆-C₂₀)-Aryl, besonders bevorzugt aus -(C₁-C₁₂)-Alkyl, Cyclohexyl und Phenyl. Am meisten bevorzugt stellen R², R⁴ jeweils -(C₁-C₁₂)-Alkyl dar. Dabei können R², R⁴ wie oben beschrieben substituiert sein. Vorzugsweise sind R², R⁴ jedoch unsubstituiert.

Vorzugsweise sind R¹, R³ jeweils unabhängig voneinander ausgewählt aus Heteroarylresten mit fünf bis zehn Ringatomen, vorzugsweise fünf oder sechs Ringatomen.

In einer Ausführungsform stellen die Reste R¹, R³ jeweils einen Heteroarylrest mit fünf Ringatomen dar.

In einer Ausführungsform sind die Reste R¹, R³ jeweils unabhängig voneinander ausgewählt aus Heteroarylresten mit sechs bis zehn Ringatomen.

In einer Ausführungsform stellen die Reste R¹, R³ jeweils einen Heteroarylrest mit sechs Ringatomen dar.

In einer Ausführungsform sind die Reste R¹, R³ ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform sind die Reste R¹, R³ ausgewählt aus Furyl, Thienyl, Pyrrolyl, Imidazolyl, Pyridyl, Pyrimidyl, Indolyl, wobei die genannten Heteroarylreste wie oben beschrieben substituiert sein können.

In einer Ausführungsform sind die Reste R¹, R³ ausgewählt aus 2-Furyl, 2-Thienyl, 2-Pyrrolyl, 2-Imidazolyl, 2-Pyridyl, 2-Pyrimidyl, 2-Indolyl, wobei die genannten Heteroarylreste wie oben beschrieben substituiert sein können.

In einer Ausführungsform sind die Reste R¹, R³ ausgewählt aus 2-Furyl, 2-Thienyl, N-Methyl-2-Pyrrolyl, N-Phenyl-2-Pyrrolyl, N-(2-Methoxyphenyl)-2-Pyrrolyl, 2-Pyrrolyl, N-Methyl-2-Imidazolyl, 2-Imidazolyl, 2-Pyridyl, 2-Pyrimidyl, N-Phenyl-2-Indolyl, 2-Indolyl, wobei die genannten Heteroarylreste nicht weiter substituiert sind.

Vorzugsweise stellen die Reste R¹, R³ Pyridyl dar, insbesondere 2-Pyridyl.

In einer Ausführungsform stellen R¹ und R³ einen Pyridylrest, bevorzugt 2-Pyridyl, dar und R² und R⁴ stehen für -(C₁-C₁₂)-Alkyl, wobei R¹, R², R³ und R⁴ jeweils wie oben beschrieben substituiert sein können.

In einer Ausführungsform sind die Reste R¹ und R³ miteinander identisch. Ebenso sind in dieser Ausführungsform die Reste R² und R⁴ miteinander identisch.

In einer Ausführungsform handelt es sich bei den erfindungsgemäßen Diastereomeren **I.1** und **I.2** um Verbindungen gemäß den Formeln (**8.1**) und (**8.2**):

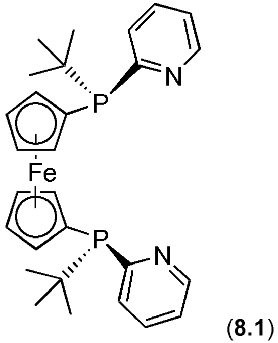

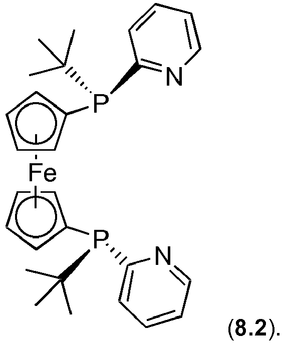

Die Erfindung betrifft weiterhin Komplexgemische umfassend einen ersten Komplex umfassend Pd und ein erfindungsgemäßes Diastereomer gemäß Formel (**I.1**) und einen zweiten Komplex umfassend Pd und ein erfindungsgemäßes Diastereomer gemäß Formel (**I.2**). Bei diesen Komplexen dienen die erfindungsgemäßen Diastereomere **I.1** und **I.2** als zweizähnige Liganden für das Metallatom. Die Komplexe dienen beispielsweise als Katalysatoren für die Alkoxycarbonylierung. Mit den erfindungsgemäßen Komplexen lassen sich hohe Ausbeuten bei der Alkoxycarbonylierung einer Vielzahl unterschiedlicher ethylenisch ungesättigter Verbindungen erzielen.

Das molare Verhältnis des ersten zum zweiten Komplex liegt vorzugsweise im Bereich von 10:90 bis 70:30, insbesondere von 20:80 bis 60:40, bevorzugt von 30:70 bis 50:50, am meisten bevorzugt von 35:65 bis 45:55.

Die erfindungsgemäßen Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Naphthochinon, Trifluoroacetat-Anionen oder Chloridanionen.

Die Erfindung betrifft weiterhin die Verwendung eines erfindungsgemäßen Diastereomerengemisches zur Katalyse einer Alkoxycarbonylierungsreaktion. Dabei können die erfindungsgemäßen Diastereomere insbesondere als erfindungsgemäßes Metallkomplexgemisch eingesetzt werden.

Die Erfindung betrifft außerdem ein Verfahren umfassend die Verfahrensschritte:
a) Vorlegen einer ethylenisch ungesättigten Verbindung;
b) Zugabe eines erfindungsgemäßen Diastereomerengemisches und einer Verbindung, welche Pd umfasst,
   oder Zugabe eines erfindungsgemäßen Komplexgemisches;
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 4 bis 30 Kohlenstoffatome, bevorzugt 6 bis 22 Kohlenstoffatome, besonders bevorzugt 8 bis 12 Kohlenstoffatome. In einer besonders bevorzugten Ausführungsform umfasst die ethylenisch ungesättigte Verbindung 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure;
Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren *n*-Butenen, Isobutan und *n*-Butan nach Abtrennung von Isobuten aus Raffinat I. Raffinat III ist ein Teil der beim Naphthacracken entstehenden C₄-Fraktion und besteht im Wesentlichen aus den isomeren *n*-Butenen und *n*-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch die erfindungsgemäßen Pd-Komplexe katalysiert. Die Pd-Komplex können dabei in Verfahrensschritt b) entweder als präformierte Komplexe umfassend Pd und das erfindungsgemäße Diastereomerengemisch zugegeben werden, oder *in situ* aus einer Verbindung, welche Pd umfasst, und dem freien Diastereomerengemisch gebildet werden. Dabei wird die Verbindung, welche Pd umfasst, auch als Katalysatorvorstufe bezeichnet.

In dem Fall, dass der Katalysator *in situ* gebildet wird, kann das Diastereomerengemisch im Überschuss zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist PdCl₂.

Der Alkohol im Verfahrensschritt c) kann verzweigt oder linear sein, cyclisch, alicyclisch, teilweise zyklisch oder aliphatisch und stellt insbesondere ein C₁- bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol. Somit sind auch ungesättigte, nicht-aromatische Alkohole zugelassen.

In einer Ausführungsform handelt es sich bei dem Alkohol um ein Alkanol mit einer oder mehrerer Hydroxylgruppen und 1 bis 30 Kohlenstoffatomen, bevorzugt 1 bis 22 Kohlenstoffatomen, besonders bevorzugt 1 bis 12 Kohlenstoffatomen, am meisten bevorzugt 1 bis 6 Kohlenstoffatomen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis des erfindungsgemäßen Diastereomerengemisches zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1. Dabei entspricht das genannte molare Verhältnis dem Verhältnis der gesamten Stoffmenge der beiden Diastereomeren **I.1** und **I.2** zu der Stoffmenge an Pd.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ≤3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beispiele

Die folgenden Beispiele veranschaulichen die Erfindung.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

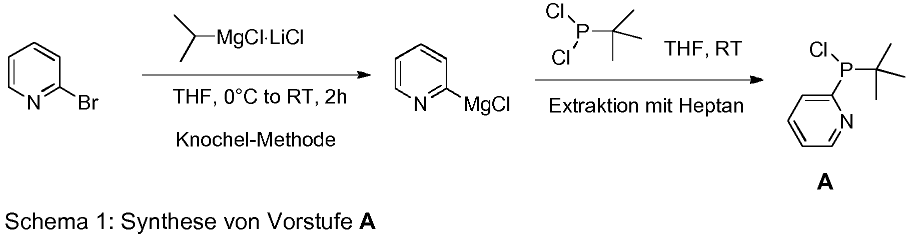

In einen 50 ml-Rundkolben mit Magnetrührer und Septum werden unter Argon 8,07 ml einer 1,3 M Isopropylmagnesium-chloridlösung (Knochel-Reagenz) gegeben und auf-15 °C gekühlt. Danach werden zügig 953.5 µl (10 mmol) 2-Bromopyridin zugetropft. Die Lösung wird sofort gelb. Man lässt auf -10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Laut GCMS hat sich viel Produkt gebildet. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).

Analytische Daten: ¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).
¹³C NMR (75 MHz, C₆D₆): δ 162,9, 162,6, 148,8, 135,5, 125,8, 125,7, 122,8, 35,3, 34,8, 25,9 und 25,8.
³¹P NMR (121 MHz, C₆D₆) δ 97,9.
MS (EI) *m*:*z* (relative Intensität) 201 (M⁺,2), 147(32), 145 (100), 109 (17), 78 (8), 57,1 (17).

### Darstellung von 1,1'-Bis(tert-butyl-2-pyridylphosphino)ferrocen (Verbindung 8)

| | |
|---|---|
| Chemikalienansatz: | 6,4 g Ferrocen (34,4 mmol) |
| | 11 ml ml TMEDA (8 g, 68,9 mmol, 2 eq) |
| | 44,1 ml 1,6N-Butyllithium (Hexan) (70,6 mmol, 2,05 eq) |
| | 12,5 ml (13,7 g, 68 mmol Chlor(tert-butyl-2-pyridyl)phosphin absolutes Heptan, absolutes Wasser, Na₂SO₄ (wasserfrei) |

In einem 250 ml Dreihalskolben versehen mit einem Tieftemperaturthermometer, einem Magnetrührer und Rückflusskühler werden 6,4 g Ferrocen unter Argon eingewogen und 70 ml absolutes Heptan zugegeben. Das Ferrocen löst sich vollständig. Danach werden 11 ml TMEDA zur Lösung gegeben und anschließend 44,1 ml 1,6 N n-BuLi zugesetzt. Die Reaktionslösung wird über Nacht bei Raumtemperatur stehen gelassen. Es bildet sich ein Feststoff (große orange Kristalle). Die überstehende Lösung wird entfernt. Zu dem Feststoff werden 100 ml Heptan gegeben, es wird mittels eines Eisbades auf ca. 5 °C gekühlt und dann werden 12,5 ml Chlor(tert-butyl-2-pyridyl)phosphin gelöst in 10 ml Heptan langsam innerhalb einer halben Stunde zugetropft. Die großen Kristalle lösen sich langsam auf und es bildet sich ein Niederschlag von Lithiumchlorid. Diese Suspension wird eine halbe Stunde bei 5 °C und dann eine Stunde bei Raumtemperatur gerührt. Die organische Phase wird dreimal mit je 20 ml entgastem Wasser gewaschen. Anschließend wird die organische Phase über Na₂SO₄ (wasserfrei) getrocknet, es wird vom Natriumsulfat abfiltriert, das Natriumsulfat wird dreimal mit jeweils 20 ml Heptan gewaschen und die vereinigte Lösung im Vakuum eingeengt. Es bildet sich ein orangenes Öl, das über Nacht im Kühlschrank durchkristallisiert. Ausbeute: 17,1 g = 96 %

### Analytische Daten:

¹H NMR (300 MHz, C₆D₆): δ 8.66- 8.56 (m, 2H, Py), 7.76-7.69 (m, 2H, Py), 7.08-6.97 (m, 2H, Py), 6.69-6.61 (m, 2H, Py), 5.17(m, 1H, Ferrocenyl), 4.94(m, 1H, Ferrocenyl), 4.37(m, 1H, Ferrocenyl), 4.17(m, 1H, Ferrocenyl), 4.05(m, 1H, Ferrocenyl), 3.98-3.93(m, 3H, Ferrocenyl), 1.14(d, *J* = 12.7 Hz, 9H, t-Bu), 1.12(d, *J* = 12.7 Hz, 9H, t-Bu).
¹³C NMR (75 MHz, C₆D₆): δ 163.6, 163.5, 149.8, 149.8, 149.6, 134.6, 134.4, 132.5, 132.4, 132.0, 132.0, 122.7, 78.4, 78.0, 77.9, 77.6, 74.2, 74.1, 74.0, 74.0, 73.8, 72.6, 72.4, 71.7, 71.6, 71.5, 31.8, 31.7, 31.7, 31.6, 28.3 und 28.2.
³¹P NMR (121 MHz, C₆D₆) δ 7.3 und 7.1

### Trennung der diasteromeren Formen von Verbindung 8

Wie an den zwei nah beieinander liegenden Phosphinsignalen bei δ 7.3 und 7.1 ppm zu erkennen ist, liegt die Verbindung **8** in zwei diastereomeren Formen vor. Diese wurde wie folgt voneinander getrennt.

Zuerst wurden von dem Diastereomerengemisch die jeweiligen Boranaddukte hergestellt und diese dann säulenchromatographisch getrennt. Dabei konnten drei Produkte isoliert werden. Die jeweiligen diastereomeren Boranaddukte und ein monosubstituiertes Nebenprodukt.

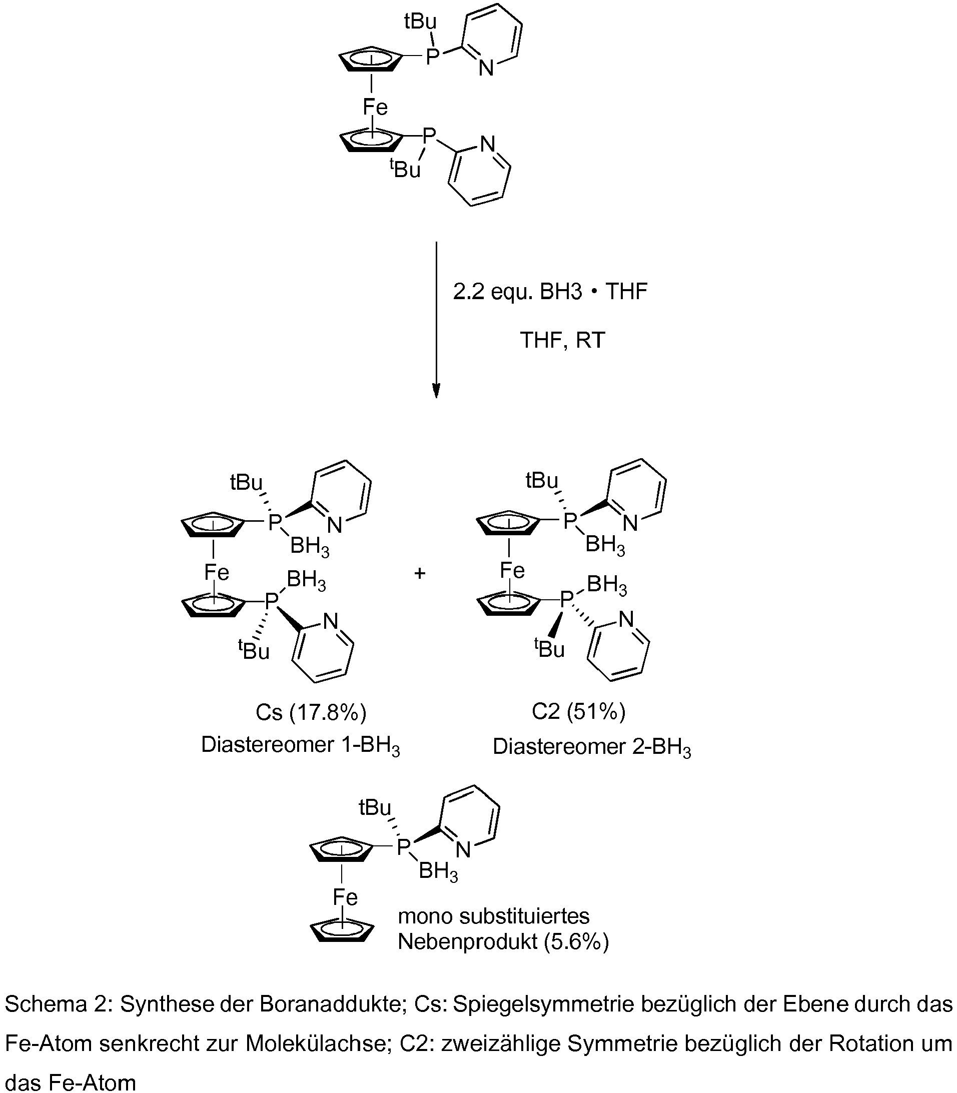

In einem 50 ml Rundkolben mit Stickstoffhahn und Magnetrührkern werden unter Argon 700 mg (1,36 mmol) des rorbraunen Bis(2-pyridyl-tert.-butylphosphino)-ferrocen Liganden vorgelegt und mit einem Septum verschlossen. Nach Zugabe von 10 ml THF hat sich eine klare orange-rote Lösung gebildet. Bei Raumtemperatur werden jetzt 2,99 ml (2,2 eq, 2,99 mmol) einer 1 M Boranlösung zügig dazugegeben. Nach 2 Tagen Rühren liegt immer noch eine klare orange-rote Lösung vor. Ein Dünnschichtchromatogramm zeigt deutlich zwei Produkte, die mit wässriger KMnO₄ Lösung angefärbt werden können. R_{f1} = 0,15, R_{f2} = 0,31, (Ethylacetat:Heptan = 1:7). Das Boranaddukt wird mit einer Combiflashapparatur (CombiFlash® Rf, TELEDYNE ISCO, A Teledyne Technologies Company) zwei Mal chromatographiert. (5 min reines Heptan, dann lässt man in 40 min den Ethylacetat-Gehalt auf 5% ansteigen). Beim ersten Durchlauf lässt sich das schnell laufende mono substituierte Boranaddukt isolieren. Ausbeute: 28 mg (5.6%). Beim zweiten Durchlauf erhält man das Diastereomere 1-BH₃ in 132 mg (17.9%) Ausbeute und das etwas langsamer laufende Diasteremer 2-BH₃ in 376 mg (51%) Ausbeute. Beide Verbindungen sind orange-braune Feststoffe.

Monosubstituiertes Nebenprodukt: ¹H NMR (300 MHz, CDCl₃): δ 8,87 (m, 1H, py), 8,30 (m, 1H, py), 7,83 (m, 1H, py), 7,43 (m, 1H, py), 5,21 (m, 1H, Ferrocenyl), 4,74 (m, 1H, Ferrocenyl), 4,43 (m, 1H, Ferrocenyl), 3,82 (s, 5H, Cp⁻), 1,01 (d, J = 14,5 Hz, 9H, tBu), 1,60-0,36 (br, BH₃).
¹³C NMR (75 MHz, CDCl₃): δ 149,4, 149,3, 135,7, 135,5, 130,5, 130,2 (Py), 75,8, 75,6, 74,1, 71,9, 71,8, 70,6, 70,4 (Ferrocenyl), 69,5 (Cp⁻), 31,5, 31,1 und 25,9 (tBu).
³¹P NMR (121 MHz, C₆D₆) δ 30,3 (m(br), P-BH₃), Ausbeute: gelbes Öl, 28 mg (5,6%).

Diastereomer 1-BH₃ (Cs): ¹H NMR (300 MHz, CDCl₃): δ 8,91 (m, 2H, py), 8,26 (m, 2H, py), 7,83 (m, 2H, py), 7,44 (m, 2H, py), 5,25 (m, 2H, Ferrocenyl), 4,24 (m, 2H, Ferrocenyl), 4,07 (m, 2H, Ferrocenyl), 3,62 (m, 2H, Ferrocenyl), 0,99 (d, J = 14,0 Hz, 18H, tBu), 1,54-0,19 (br, BH₃, schlecht aufgelöst)).
¹³C NMR (75 MHz, CDCl₃): δ 154,7, 153,7, 149,7, 149,6, 135,6, 135,4, 130,3, 130,0, 124,8, 124,7 (Py), 76,1, 75,6, 75,9, 75,2, 74,7, 74,6, 72,9, 72,7, 66,3 und 65,5 (Ferrocenyl), 31,4, 30,9, 25,8 und 25,7 (tBu)
³¹P NMR (121 MHz, C₆D₆) δ 29,9 (d (br), J = 68,1 Hz, P-BH₃), Ausbeute: 132 mg (17,9%), oranger Feststoff.

Diastereomer 2-BH₃ (C2): ¹H NMR (300 MHz, CDCl₃): δ 8,88 (m, 2H, py), 8,28 (m, 2H, py), 7,85 (m, 2H, py), 7,47 (m, 2H, py), 4,73 (m, 2H, Ferrocenyl), 4,67 (m, 2H, Ferrocenyl), 4,29 (m, 2H, Ferrocenyl), 3,57 (m, 2H, Ferrocenyl), 0,98 (d, J = 14,6 Hz, 18H, tBu), 1,61-0,25 (br, BH₃, schlecht aufgelöst)).
¹³C NMR (75 MHz, CDCl₃): δ 154,8, 153,9, 149,3, 149,2, 135,7, 135,6, 130,5, 130,2, 124,8 (Py), 76,3, 74,8, 74,7, 74,6, 73,2, 73,1, 66,1 und 65,3 (Ferrocenyl), 31,4, 31,0 und 25,8 (tBu).
³¹P NMR (121 MHz, C₆D₆) δ 30,1 (d (br), J = 63,7 Hz, P-BH₃). Ausbeute: 376 mg (51%), oranger Feststoff.

Die freien Phosphinliganden (Diastereomer 1 (Cs) **8.1** und Diastereomer 2 (C2) **8.2** können nach folgender Vorschrift aus den Boranaddukten hergestellt werden.

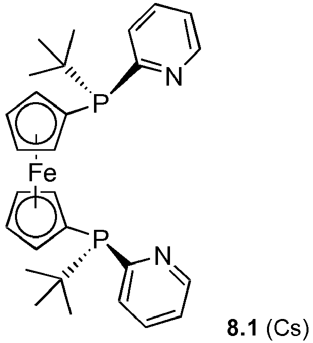

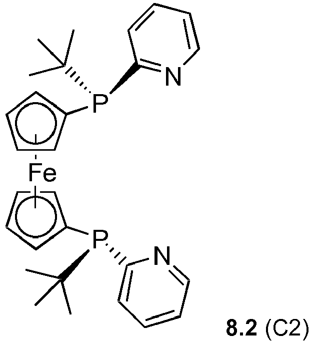

In einem sekurierten 50 ml-Rundkolben mit Magnetrührkern werden unter Argon 376 mg Diastereomer-2-BH₃ (C2) eingewogen und mit einem Septum verschlossen. Dann werden 7 ml absolutes Morpholin zugegeben und es bildet sich eine orange Suspension, die sich bei 50 °C auf dem Wasserbad langsam zu einer klaren orangen Lösung auflöst. Laut Dünnschichtchromatogramm und ³¹P-NMR hat sich nach 4 Stunden das Boranaddukt vollständig zum freien Phosphin umgesetzt. Nach Abkühlen der nun klaren orangen Lösung wird das Morpholin im Ölpumpenvakuum entfernt und der orange Rückstand wird chromatographiert. Das Chromatographieren ist notwendig, um das Produkt vom Morpholinboranaddukt abzutrennen. Zunächst wird das Laufmittel 2:1 (Heptan/Ethylacetat) von gelöstem Sauerstoff befreit, in dem man eine Stunde Argongas hindurchleitet. Ein 250 ml-Dreihalskolben mit Septum, Stickstoffansatz und einer Säule gefüllt mit Kieselgel 60 wird oben mit einem weiteren Septum verschlossen, mehrmals mit Argon sekuriert und mit dem Laufmittel eluiert. Der orange Rückstand wird in 2-3 ml Laufmittel gelöst und auf die Säule aufgetragen. Das Phosphin kann jetzt chromatographiert werden indem man via Transfernadel Laufmittel unter Argon auf die Säule gibt. Durch die orange Farbe des Produktes kann das Ende der Chromatographie leicht gesehen werden. Die chromatographierte orange Lösung wird mit einer Spritze in einen Stickstoffkolben überführt und im Hochvakuum vom Lösemittel befreit. Man erhält ein gelbes zähes Öl, das langsam fest wird. Ausbeute 312 mg (87,3%)

Diastereomer 2 (C2) **8.2,:** ¹H NMR (300 MHz, C₆D₆): δ 8,58 (m, 2H, py), 7,72 (t,t, J = 7,8 Hz, 1,3 Hz, 2H, py), 7,02 (t,t, J = 7,6 Hz, J = 2,1 Hz, 2H, py), 6,68-6,62 (m, 2H, py), 4,93 (m, 2H, Ferrocenyl), 4,37 (m, 2H, Ferrocenyl), 3,95 (m, 4H, Ferrocenyl), 1,13 (d, J = 12,0 Hz, 18H, tBu).
¹³C NMR (75 MHz, CDCl₃): δ 163,6 und 163,4 (C), 149,6, 149,5, 134,6, 134,4, 132,6, 131,9, 122,7 (py), 78,5, 77,9, 74,0, 73,9, 73,7, 72,5, 71,7, 71,5 (Ferrocenyl), 31,8 31,6, 28,3 und 28,1 (tBu).
³¹P NMR (121 MHz, C₆D₆) δ 7,1.
HRMS (ESI) m/z⁺ berechnet für C₂₈H₃₄FeN₂P₂ (M+H)⁺ 517,16197; gefunden: 517,16221.

Analog kann auch das andere Diastereomer-1 (Cs) **8.1** hergestellt werden. Hier wurden 318 mg Boranaddukt eingesetzt und man erhält nach Chromatographie 219 mg (73%) des rot orangen Diastereomer-1 (Cs) **8.1**.

Diastereomer 1 (Cs) **8.1:** ¹H NMR (300 MHz, C₆D₆): δ 8,63 (m, 2H, py), 7,72 (t,t, J = 7,8 Hz, 1,1 Hz, 2H, py), 7,04 (t,t, J = 7,6 Hz, J = 2,1 Hz, 2H, py), 6,66 (m, 2H, py), 5,17 (m, 2H, Ferrocenyl), 4,17 (m, 2H, Ferrocenyl), 4,05 (m, 2H, Ferrocenyl), 3,95 (m, 2H, Ferrocenyl), 1,11 (d, J = 12,3 Hz, 18H, tBu).
¹³C NMR (75 MHz, C₆D₆): δ 163,5 und 163,3 (C), 149,7, 149,6, 134,5, 134,3, 132,4, 131,8 und 122,6 (py), 77,9, 77,4, 74,1, 74,0, 73,8, 72,3, 71,5 und 71,4 (Ferrocenyl), 31,7, 31,5, 28,2 und 28,0 (tBu).
³¹P NMR (121 MHz, C₆D₆) δ 7,2.
HRMS (ESI) m/z⁺berechnet für C₂₈H₃₄FeN₂P₂ (M+H)⁺ 517,16197; gefunden 517,16221.

Aus der Diastereomerenmischung kann ein Isomerenverhältnis **8.2:8.1** (C2:Cs) von 56:43 (NMR-Spektren) bestimmt werden.

### Herstellung der Palladiumkomplexe K5.1 und K5.2

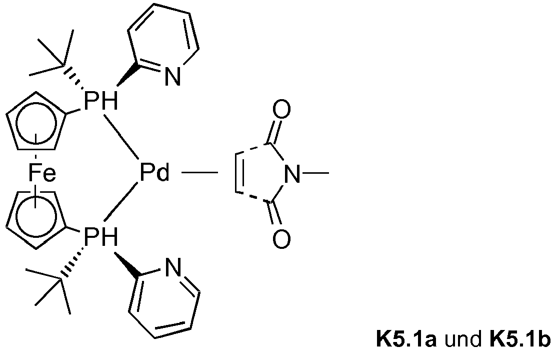

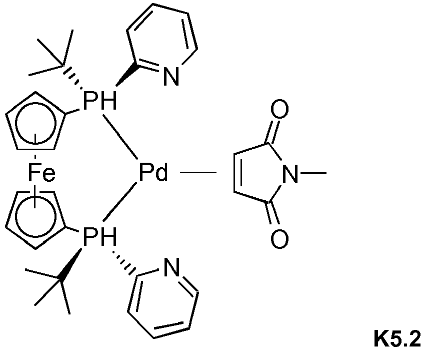

Von den diastereomeren reinen Phosphinliganden **8.1** und **8.2** werden die entsprechenen Palladiumkomplexe **K5.1a** und **K5.1b** mit Cs-Symmetrie und der Komplex **K5.2** mit C1-Symmetrie in Gegenwart von Maleinimid in Heptan wie folgt dargestellt.

Komplex **K5.2:** 58.1 mg (0.274 mmol) Palladiumprecursor (Cyclopentadienyl-allyl-palladium) werden in einem 10 mL Schlenkgefäß eingewogen und in 5 mL ausgefrorenen Heptan gelöst. Die rote klare Lösung wird über Celite in einen mit Stickstoff inertisierten 25 mL Kolben filtriert. In einem zweiten Schlenkgefäß werden unter Argon 150 mg (0.29 mmol) Diastereomer **8.2** (C2) und 30.4 mg (0.274 mmol) N-Methylmaleinimid in 6 mL Heptan gelöst. Das N-Methlymaleinimid geht erst durch Erwärmen bei 60 °C auf dem Wasserbad vollständig in Lösung. Die klare gelbe orange Lösung wird bei Raumtemperatur mit der Spritzenpumpe langsam zu der roten Palladiumprecursorlösung zugetropft. Die Lösung hellt sich dabei auf und ein gelber Niederschlag bildet sich. Am nächsten Tag lässt man den Niederschlag absetzen und dekantiert die überstehende Lösung. Nach 3 Mal waschen mit 1-2 mL Heptan wird der gelbe Niederschlag an der Ölpumpe trocken gezogen. Man erhält 200 mg (95%) eines gelben Feststoffes. Dem ³¹P-NMR zufolge muß sich aus dem C2-symmetrischen Liganden ein C1-symmetrischer Komplex gebildet haben, der die charakteristischen zwei Dubletts zeigt.
¹H NMR (300 MHz, C₆D₆): δ 8,48 (m, 2H, py), 8,12 (m, 2H, py), 7,13 (m, 1 H, py), 7,02 (t,t, J = 7,6 Hz, J = 2,3 Hz, 1H, py), 6,63 (m, 2H, py), 5,32 (m, 1H, Ferrocenyl), 4,89 (m, 1H, Ferrocenyl), 4,45 (m, 2H, Ferrocenyl), 3,95 (m, 1H, Ferrocenyl), 3,92 (m, 2H, Ferrocenyl), 3,85 (m; 2H, Ferrocenyl), 3,44 (m; 1H, Ferrocenyl), 3,03 (s, 3H, NMe), 1,36 (d, J = 14,9Hz, 9H, tBu), 1,32 (d, J = 14,6Hz, 9H, tBu).
¹³C NMR (75 MHz, C₆D₆): δ 175,9 und 175,8 (CO), 160,2, 159,7, 158,5 und 158 (C), 149,5, 149,4, 135,6, 135,4, 135,1, 135,0, 134,8, 134,5, 133,9, 124,3, 123,9 (py), 78,6, 78,3, 76,8, 76,5, 75,0, 74,8, 74,4, 74,2, 73,8, 73,4, 72,7, 72,6, 72,5, 71,0, 70,5, 70,4 (Ferrocenyl), 52,6, 52,5, 52,2, 52,1, 51,1, 51,0, 50,7, 50,6 (Maleinimide), 35,5 35,3, 35,1, 28,1, 28,0, 27,4, 27,3 (tBu), 23,5 (NMe).
³¹P NMR (121 MHz, C₆D₆) δ 47,3 (d, J = 16Hz), 46,4 (d, J = 16Hz).

Komplex **K5.1:** Die Herstellung von **K5.1** aus dem Diastereomer **8.1** erfolgt analog zur Herstellung von **K5.2.**
¹H NMR (300 MHz, C₆D₆): δ 8,27 (m, 2,77H, py), 7,74 (t, J = 7,3 Hz, 2H, py), 7,62 (m, 0,77 H, py), 6,81 (t,t, J = 7,7 Hz, J = 2,2 Hz, 2H, py), 6,66 (t,t, J = 7,7 Hz, J = 2,1 Hz, 0,77H, py), 6,39 (m, 2,77H, py), 4,66 (m, 0,77H, Methin), 4,49 (m, 2H, Methin), 4,42 (m, 0,77H, Methin), 4,33 (m, 2H, Methin), 4,27 (m; 2H, Methin I), 4,19 (m; 0,77H, Methin), 4,05 (m; 2,77H, Methin), 3,95 (m; 2,77H, Methin), 3,10 (s, 3H, NMe), 3,03 (s, 1,21H, NMe), 1,36 (d, J = 13,9Hz, 25,26H, tBu).
³¹P NMR (121 MHz, C₆D₆) δ 46,9 und 46,3. Ausbeute: 46 mg, (90%), gelber Feststoff.

Aus dem ¹H-NMR Spektren geht hervor, dass der Ligand **8.1** (Cs) zu zwei diastereomeren Cssymmetischen Palladiumkomplexen **K5.1a** und **K5.1b** (Cs) im Verhältnis 72:28 reagiert, da das Maleinimid zwei unterscheidbare Positionen einnehmen kann. Das Verhältnis kann aus den Flächenintegralen der N-Methylgruppen bei 3.10 und 3.03 ppm im ¹H-NMR bestimmt werden. Ebenso zeigt das ³¹P-NMR zwei Singuletts, die den zwei möglichen diastereomeren Komplexen mit Cs-Symmetrie zugeordnet werden können.

Der Ligand Diastereomer **8.2** (C2) dagegen führt zu einem einheitlichen Komplex mit C1-Symmetrie. Durch das fest gebundenen Maleinimid an das Metallzentrum geht zwar die C2-Symmetrie verloren, aber eine Drehung des Maleinimids um 180° würde im Gegensatz zu dem Diastereomer **8.1** (Cs) zu keinem neuen Isomer führen. Hier zeigt das Maleinimid nur ein Singulett bei 3.03 ppm im ¹H-NMR und wegen der C1-Symmetrie 2 Dubletts im ³¹P-NMR.

### Allgemeine Vorschrift zur Durchführung der Hochdruckexperimente

Allgemeine Versuchsvorschrift für Autoklavenversuche in Glasvials:
Es wird ein 300ml Parrreaktor verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit deren Ergebnissen Ausbeuten und Selektivitäten bestimmt wurden.

### Analytik

GC Analytik: Für die GC Analyse wird ein Chromatograph der Firma Agilent GC Agilent 7890A mit einer 30 m HP5-Säule verwendet. Temperaturprofil: 35 °C, 10 min; 10 °C/min zu 200 °C; das Injektionsvolumen beträgt 1 µl mit einem Split von 50:1.
Retentionszeit für iso-C9-Ester 19.502-20.439 min (Hauptpeak: 19.990 min)
Retentionszeit für n-C9-Ester: 20.669, 20.730, 20.884, 21.266 min.

### Auswertung der Versuche

Die im Folgenden angegebenen n-Selektivitäten beziehen sich auf den Anteil der endständigen Methoxycarbonylierung bezogen auf die Gesamtausbeute an Methoxycarbonylierungsprod ukten.

### Methoxycarbonylierung von 1-Octen

Um die Aktivität der Diastereomeren **K5.1** und **K5.2** des Komplexes [Pd(Cp₂Fe)(P(2-pyridyl)(*t-*butyl))₂ η²-(*N*-Methylmaleinimide)] zu untersuchen, wird Gemisch von **K5.1** und **K5.2** im Stoffmengenverhältnis 40:60 eingesetzt.

Als Benchmarkreaktion dient die Methoxycarbonylierung von 1-Octen zu Nonansäuremethylester.

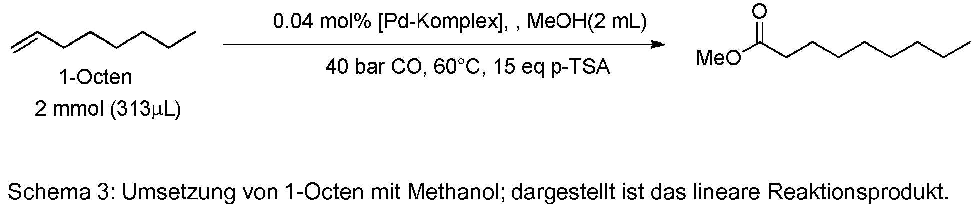

In den Versuchen werden die Reaktionsbedingungen so gewählt, dass kein vollständiger Umsatz stattfinden kann (40 bar CO, 60 °C T = variabel). Um die Versuche durchzuführen, werden 2 Stammlösungen hergestellt. Eine Stammlösung besteht aus dem Komplexgemisch (2.93 mg [Pd] in 5 mL MeOH), die andere Stammlösung besteht aus der Säure (22.8 mg para-Toluolsulfonsäure in 10 mL MeOH). Jeweils ein Milliliter Stammlösung werden in ein 4 mL Gläschen ausgestattet mit Septum, Kanüle und kleinem Magnetrührkern unter Argon zusammengegeben und in ein Karussell gestellt, das wieder in einen 300mL Parr-Autoklaven gestellt wird. Nach Spülen mit Argon und CO werden 40 bar CO aufgepresst und der Autoklav wird dann in einen Aluminiumblock, der auf 60 °C vorgeheizt ist, hineingestellt.

Drei Versuche dieser Art werden durchgeführt mit Variation der Reaktionszeiten von 15 Minuten, 30 Minuten und 40 Minuten. Nach der Reaktion wird der Autoklave auf Raumtemperatur gebracht und vorsichtig entspannt. Dann werden 300µL iso-Octan als Standard für die quantitative GC-Bestimmung zugegeben und gut durchmischt. Die Ergebnisse sind in der folgenden Tabelle zusammengestellt:

| Katalysator | Esterausbeute (%) | n-Selektivität (%) | Reaktionszeit (min) |
|---|---|---|---|
| Mischung **K5.2** und **K5.1** | 15 | 83 | 15 |
| Mischung **K5.2** und **K5.1** | 53 | 82 | 30 |
| Mischung **K5.2** und **K5.1** | 65 | 82 | 40 |

Aus Tabelle 3 geht hervor, dass das erfindungsgemäße Diastereomerengemisch sehr gute katalytische Eigenschaften für die Alkoxycarbonylierung von ethylenisch ungesättigten Verbindungen, insbesondere von langkettigen Olefinen aufweist. Somit kann auf eine Diastereomerentrennung verzichtet werden.

## Patentansprüche

1. Diastereomerengemisch umfassend Diastereomere gemäß den Formeln (**I.1**) und (**I.2**) 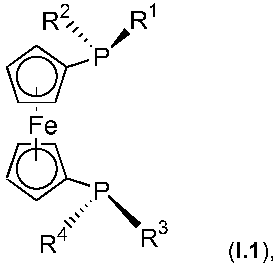 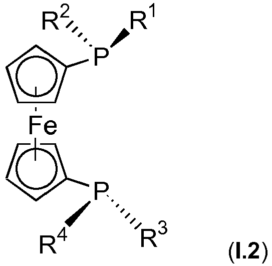 wobei
R², R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl;
die Reste R¹, R³ jeweils für einen -(C₃-C₂₀)-Heteroarylrest stehen;
R¹, R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können;
und
R², R⁴, falls sie für -(C₁-C₁₂)-Alkyl, , -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C3-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

2. Diastereomerengemisch nach Anspruch 1,
wobei das molare Verhältnis von (**I.1**) zu (**I.2**) im Bereich von 10:90 bis 70:30 liegt.

3. Diastereomerengemisch nach einem der Ansprüche 1 bis 2,
wobei R², R⁴ jeweils unabhängig voneinander ausgewählt sind aus -(C₁-C₁₂)-Alkyl, Cyclohexyl und Phenyl.

4. Diastereomerengemisch nach einem der Ansprüche 1 bis 3,
wobei R¹, R³ jeweils für einen Heteroarylrest mit fünf bis zehn Ringatomen stehen.

5. Diastereomerengemisch nach einem der Ansprüche 1 bis 4,
wobei R¹, R³ jeweils für Pyridyl stehen.

6. Diastereomerengemisch nach einem der Ansprüche 1 bis 5,
wobei R¹, R³ jeweils für identische Reste stehen und R², R⁴ jeweils für identische Reste stehen.

7. Diastereomerengemisch nach einem der Ansprüche 1 bis 6,
wobei es sich bei den Diastereomeren **I.1** bzw. **I.2** Verbindungen gemäß den Formeln (**8.1**) bzw. (**8.2**) handelt

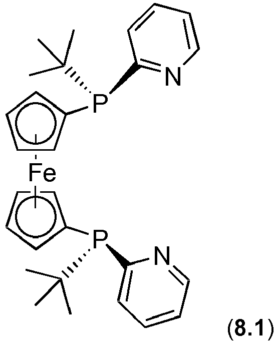

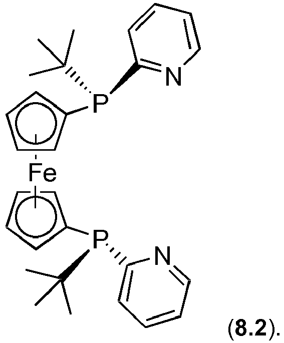

8. Komplexgemisch umfassend einen ersten Komplex umfassend Pd und ein Diastereomer gemäß Formel (**I.1**) nach einem der Ansprüche 1 bis 7 und einen zweiten Komplex umfassend Pd und ein Diastereomer gemäß Formel (**I.2**) nach einem der Ansprüche 1 bis 7.

9. Verfahren umfassend die Verfahrensschritte:
a) Vorlegen einer ethylenisch ungesättigten Verbindung;
b) Zugabe eines Diastereomerengemisches nach einem der Ansprüche 1 bis 7 und einer Verbindung, welche Pd umfasst,
oder Zugabe eines Komplexgemisches nach Anspruch 8;
c) Zugabe eines Alkohols;
d) Zuführen von CO;
e) Erwärmen des Reaktionsgemisches, wobei die ethylenisch ungesättigte Verbindung zu einem Ester umgesetzt wird.

10. Verfahren nach Anspruch 9,
wobei die ethylenisch ungesättigte Verbindung 2 bis 30 Kohlenstoffatom umfasst und optional eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten aufweist.

11. Verfahren nach einem der Ansprüche 9 bis 10,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, di-n-Buten, oder Mischungen davon.

12. Verfahren nach einem der Ansprüche 9 bis 11,
wobei die ethylenisch ungesättigte Verbindung 6 bis 22 Kohlenstoffatome umfasst.

13. Verfahren nach einem der Ansprüche 9 bis 12,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

14. Verfahren nach einem der Ansprüche 9 bis 13,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

15. Verwendung einer Verbindung gemäß einem der Ansprüche 1 bis 7 oder eines Komplexes gemäß Anspruch 8 zur Katalyse einer Alkoxycarbonylierungsreaktion.

## Claims

1. Diastereomer mixture comprising diastereomers of the formulae (**I.1**) and (**I.2**) 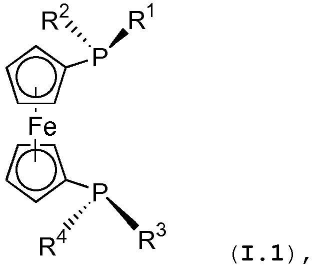 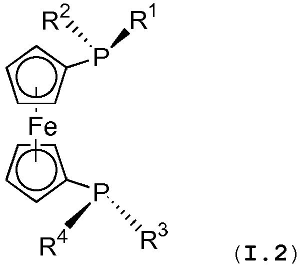 where
R², R⁴ are each independently selected from -(C₁-C₁₂)-alkyl, -(C₃-C₁₂)-cycloalkyl, -(C₃-C₁₂)-heterocycloalkyl, - (C₆-C₂₀)-aryl;
the R¹, R³ radicals are each a -(C₃-C₂₀)-heteroaryl radical;
R¹, R³ may each independently be substituted by one or more substituents selected from -(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl-(C₆-C₂₀)-aryl, -O-(C₃-C₁₂)-cycloalkyl, -S-(C₁-C₁₂)-alkyl, -S-(C₃-C₁₂)-cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀) -aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen; and
R², R⁴, if they are (C₁-C₁₂)-alkyl, -(C₃-C₁₂)-cycloalkyl, -(C₃-C₁₂)-heterocycloalkyl or - (C₆-C₂₀)-aryl, may each independently be substituted by one or more substituents selected from -(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl-(C₆-C₂₀)-aryl, -O-(C₃-C₁₂)-cycloalkyl, -S-(C₁-C₁₂)-alkyl, -S-(C₃-C₁₂)-cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀)-aryl,-(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen.

2. Diastereomer mixture according to claim 1,
where the molar ratio of (**I.1**) to (**I.2**) is in the range from 10:90 to 70:30.

3. Diastereomer mixture according to either of Claims 1 and 2,
where R², R⁴ are each independently selected from-(C₁-C₁₂)-alkyl, cyclohexyl and phenyl.

4. Diastereomer mixture according to any of Claims 1 to 3,
where R¹, R³ are each a heteroaryl radical having five to ten ring atoms.

5. Diastereomer mixture according to any of Claims 1 to 4,
where R¹, R³ are each pyridyl.

6. Diastereomer mixture according to any of Claims 1 to 5,
where R¹, R³ are each identical radicals and R², R⁴ are each identical radicals.

7. Diastereomer mixture according to any of Claims 1 to 6,
where the diastereomers **I.1** and **I.2** are compounds of the formula (**8.1**) and (**8.2**)

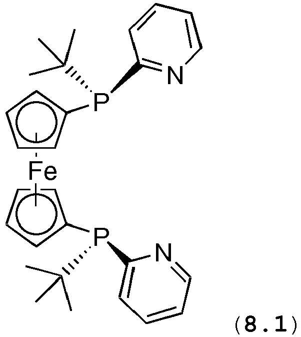

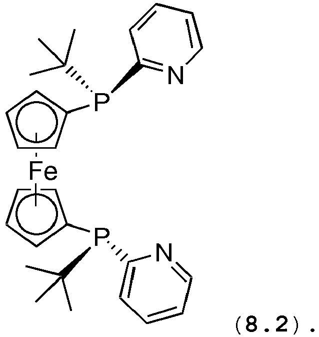

8. Complex mixture comprising a first complex comprising Pd and a diastereomer of formula (**I.1**) according to any of Claims 1 to 7 and a second complex comprising Pd and a diastereomer of formula (**I.2**) according to any of Claims 1 to 7.

9. Process comprising the following process steps:
a) initially charging an ethylenically unsaturated compound;
b) adding a diastereomer mixture according to any of Claims 1 to 7 and a compound comprising Pd, or adding a complex mixture according to Claim 8;
c) adding an alcohol;
d) feeding in CO;
e) heating the reaction mixture, with conversion of the ethylenically unsaturated compound to an ester.

10. Process according to Claim 9,
wherein the ethylenically unsaturated compound comprises 2 to 30 carbon atoms and optionally one or more functional groups selected from carboxyl, thiocarboxyl, sulpho, sulphinyl, carboxylic anhydride, imide, carboxylic ester, sulphonic ester, carbamoyl, sulphamoyl, cyano, carbonyl, carbonothioyl, hydroxyl, sulphhydryl, amino, ether, thioether, aryl, heteroaryl or silyl groups and/or halogen substituents.

11. Process according to either of Claims 9 and 10,
wherein the ethylenically unsaturated compound is selected from ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, and mixtures thereof.

12. Process according to any of Claims 9 to 11,
wherein the ethylenically unsaturated compound comprises 6 to 22 carbon atoms.

13. Process according to any of Claims 9 to 12,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

14. Process according to any of Claims 9 to 13,
wherein the alcohol in process step c) is selected from methanol, ethanol, 1-propanol, 1-butanol, 1-pentanol, 1-hexanol, 2-propanol, *tert*-butanol, 3-pentanol, cyclohexanol, phenol, and mixtures thereof.

15. Use of a compound according to any of Claims 1 to 7 or of a complex according to Claim 8 for catalysis of an alkoxycarbonylation reaction.

## Revendications

1. Mélange de diastéréomères comprenant des diastéréomères selon les formules (I.1) et (I.2) 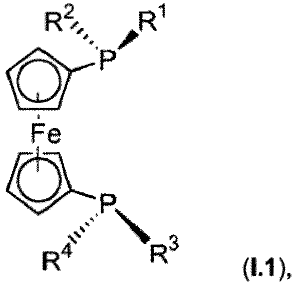 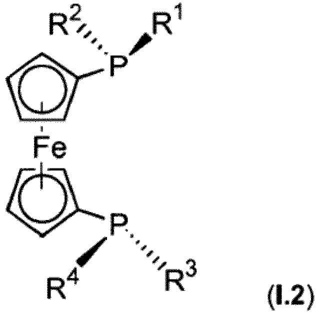 dans lesquelles
R², R⁴ sont chacun choisis indépendamment l'un de l'autre parmi alkyle en (C₁-C₁₂), cycloalkyle en (C₃-C₁₂), hétérocycloalkyle en (C₃-C₁₂), aryle en (C₆-C₂₀) ;
les radicaux R¹, R³ représentent chacun un radical hétéroaryle en (C₃-C₂₀) ;
R¹, R³ peuvent chacun être substitués indépendamment l'un de l'autre avec un ou plusieurs substituants choisis parmi alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂),-CONH-alkyle en (C₁-C₁₂), -CONH-cycloalkyle en (C₃-C₁₂),-CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀) -O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀) -O-alkyle en (C₁-C₁₂),-COOH, -OH, -SO₃H, -NH₂, halogène ;
et
R², R⁴, s'ils représentent alkyle en (C₁-C₁₂), cycloalkyle en (C₃-C₁₂), hétérocycloalkyle en (C₃-C₁₂) ou aryle en (C₆-C₂₀), peuvent chacun être substitués indépendamment l'un de l'autre avec un ou plusieurs substituants choisis parmi alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂),-CONH-alkyle en (C₁-C₁₂), -CONH-cycloalkyle en (C₃-C₁₂),-CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀) -O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀) -O-alkyle en (C₁-C₁₂),-COOH, -OH, -SO₃H, -NH₂, halogène.

2. Mélange de diastéréomères selon la revendication 1, dans lequel le rapport molaire entre (I.1) et (I.2) se situe dans la plage allant de 10:90 à 70:30.

3. Mélange de diastéréomères selon l'une quelconque des revendications 1 à 2, dans lequel R², R⁴ sont chacun choisis indépendamment l'un de l'autre parmi alkyle en (C₁-C₁₂), cyclohexyle et phényle.

4. Mélange de diastéréomères selon l'une quelconque des revendications 1 à 3, dans lequel R¹, R³ représentent chacun un radical hétéroaryle contenant cinq à dix atomes de cycle.

5. Mélange de diastéréomères selon l'une quelconque des revendications 1 à 4, dans lequel R¹, R³ représentent chacun pyridyle.

6. Mélange de diastéréomères selon l'une quelconque des revendications 1 à 5, dans lequel R¹, R³ représentent chacun des radicaux identiques, et R², R⁴ représentent chacun des radicaux identiques.

7. Mélange de diastéréomères selon l'une quelconque des revendications 1 à 6, dans lequel les diastéréomères I.1 et I.2 sont des composés selon les formules (8.1) et (8.2)

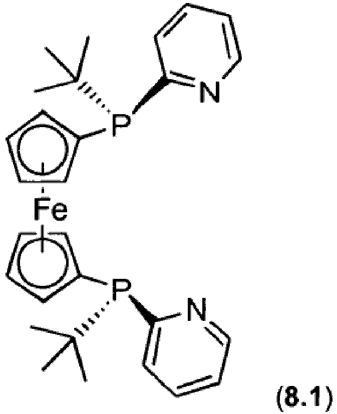

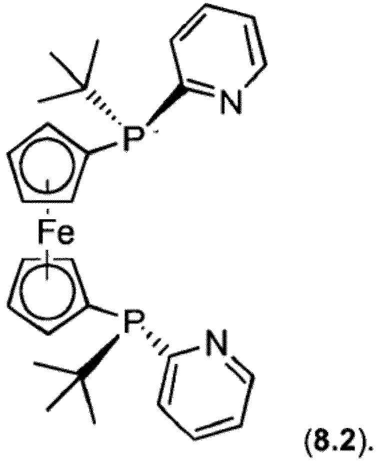

8. Mélange de complexes comprenant un premier complexe comprenant Pd et un diastéréomère selon la formule (I.1) selon l'une quelconque des revendications 1 à 7, et un deuxième complexe comprenant Pd et un diastéréomère selon la formule (I.2) selon l'une quelconque des revendications 1 à 7.

9. Procédé comprenant les étapes de procédé suivantes :
a) le chargement d'un composé éthyléniquement insaturé ;
b) l'ajout d'un mélange de diastéréomères selon l'une quelconque des revendications 1 à 7 et d'un composé qui comprend Pd,
ou l'ajout d'un mélange de complexes selon la revendication 8 ;
c) l'ajout d'un alcool ;
d) l'introduction de CO ;
e) le chauffage du mélange réactionnel, le composé éthyléniquement insaturé étant transformé en un ester.

10. Procédé selon la revendication 9, dans lequel le composé éthyléniquement insaturé comprend 2 à 30 atomes de carbone et éventuellement un ou plusieurs groupes fonctionnels choisis parmi les groupes carboxyle, thiocarboxyle, sulfo, sulfinyle, anhydride d'acide carboxylique, imide, ester d'acide carboxylique, ester d'acide sulfonique, carbamoyle, sulfamoyle, cyano, carbonyle, carbononothioyle, hydroxyle, sulfhydryle, amino, éther, thioéther, aryle, hétéroaryle ou silyle et/ou les substituants halogène.

11. Procédé selon l'une quelconque des revendications 9 à 10, dans lequel le composé éthyléniquement insaturé est choisi parmi l'éthène, le propène, le 1-butène, le *cis-*et/ou *trans*-2-butène, l'iso-butène, le 1,3-butadiène, le 1-pentène, le *cis-* et/ou *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

12. Procédé selon l'une quelconque des revendications 9 à 11, dans lequel le composé éthyléniquement insaturé comprend 6 à 22 atomes de carbone.

13. Procédé selon l'une quelconque des revendications 9 à 12, dans lequel le composé qui comprend Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

14. Procédé selon l'une quelconque des revendications 9 à 13, dans lequel l'alcool à l'étape de procédé c) est choisi parmi le méthanol, l'éthanol, le 1-propanol, le 1-butanol, le 1-pentanol, le 1-hexanol, le 2-propanol, le *tert*-butanol, le 3-pentanol, le cyclohexanol, le phénol ou leurs mélanges.

15. Utilisation d'un composé selon l'une quelconque des revendications 1 à 7 ou d'un complexe selon la revendication 8 pour la catalyse d'une réaction d'alcoxycarbonylation.
